(19) Europäisches Patentamt
European Patent Office
Office européen des brevets

(11) Veröffentlichungsnummer: **0 068 310**
**B1**

(12) # EUROPÄISCHE PATENTSCHRIFT

(45) Veröffentlichungstag der Patentschrift: **08.08.84**

(51) Int. Cl.³: **C 07 D 403/04**, C 07 D 401/04, A 61 K 31/50

(21) Anmeldenummer: **82105281.8**

(22) Anmeldetag: **16.06.82**

(54) **Neue 2-Aryl-3,4-diaza-bicyclo(4.n.0)alken-(2)-one-(5), Verfahren zu ihrer Herstellung und diese Verbindungen enthaltende Arzneimittel.**

(30) Priorität: **24.06.81 DE 3124699**

(43) Veröffentlichungstag der Anmeldung: **05.01.83 Patentblatt 83/1**

(45) Bekanntmachung des Hinweises auf die Patenterteilung: **08.08.84 Patentblatt 84/32**

(84) Benannte Vertragsstaaten: **AT BE CH DE FR GB IT LI LU NL SE**

(56) Entgegenhaltungen:
**EP - A - 0 010 156**
**DE - A - 2 845 220**
**DE - A - 2 854 475**
**US - A - 3 931 176**

(73) Patentinhaber: **BASF Aktiengesellschaft, Carl-Bosch-Strasse 38, D-6700 Ludwigshafen (DE)**

(72) Erfinder: **Rossy, Phillip A., Dr., Van-Leyden-Strasse 17, D-6700 Ludwigshafen (DE)**
Erfinder: **Thyes, Marco, Dr., Thorwaldsenstrasse 1, D-6700 Ludwigshafen (DE)**
Erfinder: **Franke, Albrecht, Dr., Mandelring 11, D-6706 Wachenheim (DE)**
Erfinder: **Gries, Josef, Dr., Roemerweg 43, D-6706 Wachenheim (DE)**
Erfinder: **Lehmann, Hans Dieter, Dr., Im Hefen 15, D-6945 Hirschberg (DE)**
Erfinder: **Koenig, Horst, Dr., Pariser Strasse 4, D-6700 Ludwigshafen (DE)**
Erfinder: **Lenke, Dieter, Dr., Kekuleplatz 1, D-6700 Ludwigshafen (DE)**

**EP 0 068 310 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue 2-Aryl-3,4-diaza-bicyclo-[4.1.0]hepten-(2)-one-(5), 2-Aryl-3,4-diazabicyclo[4.2.0]-octen-(2)-one-(5) und 2-Aryl-3,4-diaza-bicyclo[4.3.0]-nonen-(2)-one-(5), Verfahren zu ihrer Herstellung, diese Verbindungen enthaltende pharmazeutische Zubereitungen und ihre Verwendung bei der Prophylaxe und Therapie thrombo-embolischer Erkrankungen und bei hohem Blutdruck.

In der DE-OS 2 854 475 werden 2-(Acylamino)phenyl-3,4-diaza-bicyclo[4.1.0]hepten-(2)-one-(5) bei der Behandlung von thrombo-embolischen Erkrankungen und zu hohen Blutdrucks vorgeschlagen. In 4-Stellung durch eine Hydroxyalkylgruppe substituierte 2-Aryl-3,4-diaza-bicyclo[4.2.0]-octen-(2)-on-(5) werden in der US-PS 3 931 176 beschrieben. Für diese Verbindungen sind zentraldämpfende Eigenschaften erwähnt. Bekannt ist ferner das unsubstituierte 2-Phenyl-3,4-diaza-bicyclo[4.2.0]octen-(2)-on-(5) (Chem. Ber. 99 1229 (1966)), aber über pharmakologische Wirkungen dieser Verbindung ist nichts angegeben.

6-Acylaminophenyl-4,5-dihydro-3(2H)-pyridazinone sind bereits mehrfach beschrieben worden, beispielsweise werden in der DE-OS 1 670 158 in den Stellungen 4 und 5 unsubstituierte 6-(Acylamino)phenyl-4,5-dihydro-3(2H)-pyridazinone mit blutdrucksenkenden und entzündungshemmenden Eigenschaften beschrieben. Für 6-Phenyl-4,5-dihydro-3(2H)-pyridazinone, die in 4-Stellung eine Alkylgruppe tragen und im Phenylrest in p-Stellung durch eine Gruppe der Formel $-NHR^3$, in der $R^3$ beispielsweise für einen Acylrest oder einen Ethoxycarbonylrest steht, substituiert sind, werden in der DE-OS 2 304 977 cardiovasculäre und antiphlogistische Eigenschaften genannt. In der DE-OS 2 150 436 und den US-PS 3 824 271 und 3 888 901 werden blutdrucksenkend wirkende, in 5-Stellung durch einen Alkylrest substituierte 6-(Alkanoylamino)phenyl-4,5-dihydro-3(2H)-pyridazinone beschrieben. Weiterhin sind aus der DE-OS 2 727 481 und der DE-OS 2 854 191 6-(p-Alkanoylaminophenyl)-4,5-dihydro-3(2H)-pyridazinone, die in der Alkanoylgruppe durch ein oder mehrere Halogenatome substituiert sind, als Arzneimittel wegen ihren thrombozytenaggregationshemmenden und blutdrucksenkenden Eigenschaften bekannt.

In der DE-OS 2 123 246 werden blutdrucksenkend, coronarerweiternd und antiinflammatorisch wirkende 6-(p-Alkanoylaminophenyl)-4,5-dihydro-3(2H)-pyridazinone, die im Alkanoylrest eine substituierte Aminogruppe tragen, beschrieben. Für 6-Phenyl-4,5-dihydro-3(2H)-pyridazinone, die im Phenylrest in p-Stellung durch eine Gruppe der Formel $-NHCONR^1R^2$, in der die Reste $R^1$ und $R^2$ gleich oder verschieden sind und beispielsweise für Wasserstoff, eine Alkylgruppe oder eine Arylgruppe stehen, substituiert sind, werden in der DE-OS 2 157 453 cardiovasculäre und antiinflammatorische Eigenschaften angegeben. Ferner werden in der japanischen Patentanmeldung 53 124-279 antiallergisch, membranstabilisierend und thrombozytenaggregationshemmend wirkende 6-p-(Alkoxycarbonylaminoalkyl)-phenyl-4,5-dihydro-3(2H)-pyridazinone beschrieben.

Schließlich ist aus der DE-OS 2 845 220 bekannt, daß 5-(4,5-Dihydro-3(2H)-pyridazinon-6-yl)-indolin-2-one und 6-(4,5-Dihydro-3(2H)-pyridazinon-6-yl)-1,2,3,4-tetrahydrochinolin-2-one die Aggregation der Thrombocyten hemmen und den Blutdruck senken.

Es wurde nun gefunden, daß Diaza-bicyclo[4.n.0]alkenone der allgemeinen Formel I

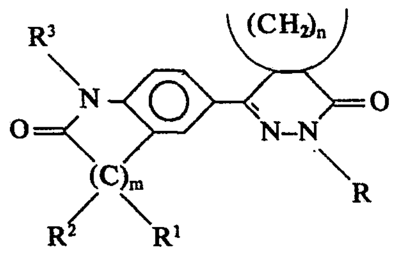

(I)

worin

m und n gleich oder verschieden sind und die Zahlen 1, 2 oder 3 bedeuten, und

R, $R^1$, $R^2$ und $R^3$ gleich oder verschieden sind und Wasserstoffatome oder Alkylgruppen mit 1 bis 6 C-Atomen bedeuten,

wertvolle pharmakolische Eigenschaften aufweisen.

Die erfindungsgemäßen Verbindungen der Formel I werden hergestellt, indem man eine Cycloalkancarbonsäure der Formel II

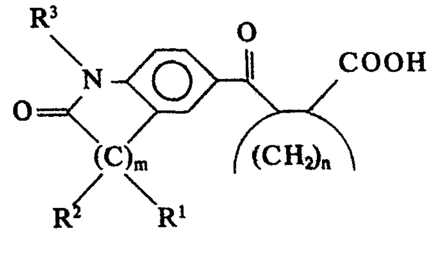

(II)

in der $R^1$, $R^2$ und $R^3$ die oben angegebenen Bedeutungen haben, mit einem Hydrazin der Formel $NH_2NHR$, in der R die oben angegebene Bedeutung hat, umsetzt.

Die Cyclisierung einer Verbindung der Formel II mit einem Hydrazin der Formel $NH_2NHR$, in der R die oben angegebenen Bedeutungen hat, zu einem Diaza-bicyclo[4.n.0]alkenon der Formel I erfolgt vorteilhaft in einem unter den Reaktionsbedingungen inerten Lösungsmittel, insbesondere einem niederen Alkohol wie Methanol, Ethanol oder Propanol, einem cyclischen aliphatischen Ether wie Tetrahydrofuran oder Dioxan, oder einem Dialkylformamid, wie Dimethylformamid, und bei Temperaturen von 40 bis 150° C, vorzugsweise 60 bis 120° C. In der Regel werden hierbei je 1 Mol der Verbindung der Formel II und 1 bis 1,2 Mol des Hydrazins verwendet.

Die Ausgangsverbindungen der Formel II werden durch Umsetzung einer Verbindung der Formel III

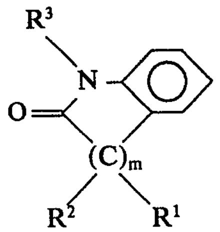

(III)

in der $R^1$, $R^2$, $R^3$ und m die oben angegebenen Bedeutungen haben, mit 1,2-Cyclopropan-, 1,2-Cyclobutan- oder 1,2-Cyclopentan-dicarbonsäureanhydrid in Gegenwart von Aluminiumchlorid unter den Bedingungen einer Friedel-Crafts-Acylierung erhalten.

Diese Acylierung kann in einem Lösungsmittel, beispielsweise Schwefelkohlenstoff, bei Temperaturen von 25 bis 150° C durchgeführt werden. Sie kann auch in einer Dimethylformamid/Aluminiumchlorid-Schmelze bei Temperaturen zwischen 50 bis 200° C, vorzugsweise 100 bis 160° C, erfolgen. Dabei ist es zweckmäßig, auf 1 Mol 1,2-Cyclopropan-, 1,2-Cyclobutan- oder 1,2-Cyclopentan-dicarbonsäureanhydrid bzw. 1 Mol einer Verbindung der Formel II, etwa 10 Mol Aluminiumchlorid und etwa 2,5 Mol Dimethylformamid zu verwenden.

Nach den genannten Verfahren werden beispielsweise die folgenden erfindungsgemäßen Verbindungen erhalten:

2-(Indolin-2-on-5-yl)-3,4-diaza-bicyclo[4.3.0]-nonen-(2)-on-(5)
2-(1-Methylindolin-2-on-5-yl)-3,4-diaza-bicyclo[4.3.0]-nonen-(2)-on-(5)
2-(1-Ethylindolin-2-on-5-yl)-3,4-diaza-bicyclo[4.3.0]-nonen-(2)-on-(5)
2-(1-Propylindolin-2-on-5-yl)-3,4-diaza-bicyclo[4.3.0]-nonen-(2)-on-(5)
2-(3-Methylindolin-2-on-5-yl)-3,4-diaza-bicyclo[4.3.0]-nonen-(2)-on-(5)
2-(3,3-Dimethylindolin-2-on-5-yl)-3,4-diaza-bicyclo[4.3.0]-nonen-(2)-on-(5)
2-(3-Ethylindolin-2-on-5-yl)-3,4-diaza-bicyclo[4.3.0]-nonen-(2)-on-(5)
2-(3,3-Diethylindolin-2-on-5-yl)-3,4-diaza-bicyclo[4.3.0]-nonen-(2)-on-(5)
2-(3-Ethyl-3-methylindolin-2-on-5-yl)-3,4-diaza-bicyclo[4.3.0]-nonen-(2)-on-(5)
2-(1,3-Dimethylindolin-2-on-5-yl)-3,4-diaza-bicyclo[4.3.0]-nonen-(2)-on-(5)
2-(1,3,3-Trimethylindolin-2-on-5-yl)-3,4-diaza-bicyclo[4.3.0]-nonen-(2)-on-(5)
2-(Indolin-2-on-5-yl)-3,4-diaza-bicyclo[4.3.0]-nonen-(2)-on-(5)
2-(Indolin-2-on-5-yl)-3,4-diaza-bicyclo[4.2.0]-octen-(2)-on-(5)
2-(1-Methylindolin-2-on-5-yl)-3,4-diaza-bicyclo[4.2.0]-octen-(2)-on-(5)
2-(1-Ethylindolin-2-on-5-yl)-3,4-diaza-bicyclo[4.2.0]-octen-(2)-on-(5)
2-(1-Propylindolin-2-on-5-yl)-3,4-diaza-bicyclo[4.2.0]-octen-(2)-on-(5)
2-(3-Methylindolin-2-on-5-yl)-3,4-diaza-bicyclo[4.2.0]-octen-(2)-on-(5)
2-(3,3-Dimethylindolin-2-on-5-yl)-3,4-diaza-bicyclo[4.2.0]-octen-(2)-on-(5)
2-(3-Ethylindolin-2-on-5-yl)-3,4-diaza-bicyclo[4.2.0]-octen-(2)-on-(5)
2-(3,3-Diethylindolin-2-on-5-yl)-3,4-diaza-bicyclo[4.2.0]-octen-(2)-on-(5)
2-(3-Ethyl-3-methylindolin-2-on-5-yl)-3,4-diaza-bicyclo[4.2.0]-octen-(2)-on-(5)
2-(1,3-Dimethylindolin-2-on-5-yl)-3,4-diaza-bicyclo[4.2.0]-octen-(2)-on-(5)
2-(1,3,3-Trimethylindolin-2-on-5-yl)-3,4-bicyclo[4.2.0]-octen-(2)-on-(5)
2-(Indolin-2-on-5-yl)-4-methyl-3,4-diaza-bicyclo[4.2.0]-octen-(2)-on-(5)
2-(Indolin-2-on-5-yl)-3,4-diaza-bicyclo[4.1.0]-hepten-(2)-on-(5)
2-(1-Methylindolin-2-on-5-yl)-3,4-diaza-bicyclo[4.1.0]-hepten-(2)-on-(5)
2-(1-Ethylindolin-2-on-5-yl)-3,4-diaza-bicyclo[4.1.0]-hepten-(2)-on-(5)
2-(1-Propylindolin-2-on-5-yl)-3,4-diaza-bicyclo[4.1.0]-hepten-(2)-on-(5)
2-(3-Methylindolin-2-on-5-yl)-3,4-diaza-bicyclo[4.1.0]-hepten-(2)-on-(5)
2-(3,3-Dimethylindolin-2-on-5-yl)-3,4-diaza-bicyclo[4.1.0]-2-(3-Ethylindolin-2-on-5-yl)-3,4-diaza-bicyclo[4.1.0]-hepten-(2)-on-(5)
2-(3,3-Diethylindolin-2-on-5-yl)-3,4-diaza-bicyclo[4.1.0]-hepten-(2)-on-(5)
2-(3-Ethyl-3-methylindolin-2-on-5-yl)-3,4-diaza-bicyclo[4.1.0]-hepten-(2)-on-(5)

2-(1,3-Dimethylindolin-2-on-5-yl)-3,4-diaza-bicyclo[4.1.0]-hepten-(2)-on-(5)
2-(1,3,3-Trimethylindolin-2-on-5-yl)-3,4-diaza-bicyclo[4.1.0]-hepten-(2)-on-(5)
2-(Indolin-2-on-5-yl)-4-methyl-3,4-diaza-bicyclo[4.1.0]-hepten-(2)-on-(5)
2-(1-Methyl-indolin-2-on-5-yl)-4-methyl-3,4-diaza-bicyclo[4.1.0]-hepten-(2)-on-(5)
2-(1,3-Dimethyl-indolin-2-on-5-yl)-4-methyl-3,4-diaza-bicyclo[4.1.0]-hepten-(2)-on-(5)
2-(1,2,3,4-Tetrahydrochinolin-2-on-6-yl)-3,4-diaza-bicyclo[4.3.0]nonen-(2)-on-(5)
2-(1-Methyl-1,2,3,4-tetrahydrochinolin-2-on-6-yl)-3,4-diaza-bicyclo[4.3.0]nonen-(2)-on-(5)
2-(1-Ethyl-1,2,3,4-tetrahydrochinolin-2-on-6-yl)-3,4-diaza-bicyclo[4.3.0]nonen-(2)-on-(5)
2-(1-Propyl-1,2,3,4-tetrahydrochinolin-2-on-6-yl)-3,4-diaza-bicyclo[4.3.0]nonen-(2)-on-(5)
2-(3-Methyl-1,2,3,4-tetrahydrochinolin-2-on-6-yl)-3,4-diaza-bicyclo[4.3.0]nonen-(2)-on-(5)
2-(3-Ethyl-1,2,3,4-tetrahydrochinolin-2-on-6-yl)-3,4-diaza-bicyclo[4.3.0]nonen-(2)-on-(5)
2-(4-Methyl-1,2,3,4-tetrahydrochinolin-2-on-6-yl)-3,4-diaza-bicyclo[4.3.0]nonen-(2)-on-(5)
2-(4-Ethyl-1,2,3,4-tetrahydrochinolin-2-on-6-yl)-3,4-diaza-bicyclo[4.3.0]nonen-(2)-on-(5)
2-(3,3-Dimethyl-1,2,3,4-tetrahydrochinolin-2-on-6-yl)-3,4-diaza-bicyclo[4.3.0]nonen-(2)-on-(5)
2-(3,3-Diethyl-1,2,3,4-tetrahydrochinolin-2-on-6-yl)-3,4-diaza-bicyclo[4.3.0]nonen-(2)-on-(5)
2-(3,4-Dimethyl-1,2,3,4-tetrahydrochinolin-2-on-6-yl)-3,4-diaza-bicyclo[4.3.0]nonen-(2)-on-(5)
2-(3,4-Diethyl-1,2,3,4-tetrahydrochinolin-2-on-6-yl)-3,4-diaza-bicyclo[4.3.0]nonen-(2)-on-(5)
2-(3-Ethyl-4-methyl-1,2,3,4-tetrahydrochinolin-2-on-6-yl)-3,4-diaza-bicyclo[4.3.0]nonen-(2)-on-(5)
2-(4-Ethyl-3-methyl-1,2,3,4-tetrahydrochinolin-2-on-6-yl)-3,4-diaza-bicyclo[4.3.0]nonen-(2)-on-(5)
2-(3,3,4,4-Tetramethyl-1,2,3,4-tetrahydrochinolin-2-on-6-yl)-3,4-diaza-bicyclo[4.3.0]nonen-(2)-on-(5)
2-(3,4-Diethyl-3,4-dimethyl-1,2,3,4-tetrahydrochinolin-2-on-6-yl)-3,4-diaza-bicyclo[4.3.0]nonen-(2)-on-(5)
2-(1,3-Dimethyl-1,2,3,4-tetrahydrochinolin-2-on-6-yl)-3,4-diaza-bicyclo[4.3.0]nonen-(2)-on-(5)
2-(1,3,4-Trimethyl-1,2,3,4-tetrahydrochinolin-2-on-6-yl)-3,4-diaza-bicyclo[4.3.0]nonen-(2)-on-(5)
2-1,3,3-Trimethyl-1,2,3,4-tetrahydrochinolin-2-on-6-yl)-3,4-diaza-bicyclo[4.3.0]nonen-(2)-on-(5)
2-(1,4,4-Trimethyl-1,2,3,4-tetrahydrochinolin-2-on-6-yl)-3,4-diaza-bicyclo[4.3.0]nonen-(2)-on-(5)
2-(1,2,3,4-Tetrahydrochinolin-2-on-6-yl)-4-methyl-3,4-diaza-bicyclo[4.3.0]nonen-(2)-on-(5)
2-(1-Methyl-1,2,3,4-tetrahydrochinolin-2-on-6-yl)-4-methyl-3,4-diaza-bicyclo-[4.3.0]nonen-(2)-on-(5)
2-(1,3-Dimethyl-1,2,3,4-tetrahydrochinolin-2-on-6-yl)-4-methyl-3,4-diaza-bicyclo[4.3.0]nonen-(2)-on-(5)
2-(1,3,4-Trimethyl-1,2,3,4-tetrahydrochinolin-2-on-6-yl)-4-methyl-3,4-diaza-bicyclo[4.3.0]nonen-(2)-on-(5)
2-(1,2,3,4-Tetrahydrochinolin-2-on-6-yl)-3,4-diaza-bicyclo[4.2.0]octen-(2)-on-(5)
2-(1-Methyl-1,2,3,4-tetrahydrochinolin-2-on-6-yl)-3,4-diaza-bicyclo[4.2.0]octen-(2)-on-(5)
1-(1-Ethyl-1,2,3,4-tetrahydrochinolin-2-on-6-yl)-3,4-diaza-bicyclo[4.2.0]octen-(2)-on-(5)
2-(1-Propyl-1,2,3,4-tetrahydrochinolin-2-on-6-yl)-3,4-diaza-bicyclo[4.2.0]octen-(2)-on-(5)
2-(3-Methyl-1,2,3,4-tetrahydrochinolin-2-on-6-yl)-3,4-diaza-bicyclo[4.2.0]octen-(2)-on-(5)
2-(3-Ethyl-1,2,3,4-tetrahydrochinolin-2-on-6-yl)-3,4-diaza-bicyclo-[4.2.0]octen-(2)-on-(5)
2-(4-Methyl-1,2,3,4-tetrahydrochinolin-2-on-6-yl)-3,4-diaza-bicyclo[4.2.0]octen-(2)-on-(5)
2-(4-Ethyl-1,2,3,4-tetrahydrochinolin-2-on-6-yl)-3,4-diaza-bicyclo[4.2.0]octen-(2)-on-(5)
2-(3,3-Dimethyl-1,2,3,4-tetrahydrochinolin-2-on-6-yl)-3,4-diaza-bicyclo[4.2.0]octen-(2)-on-(5)
2-(3,3-Diethyl-1,2,3,4-tetrahydrochinolin-2-on-6-yl)-3,4-diaza-bicyclo[4.2.0]octen-(2)-on-(5)
2-(3,4-Dimethyl-1,2,3,4-tetrahydrochinolin-2-on-6-yl)-3,4-diaza-bicyclo[4.2.0]octen-(2)-on-(5)
2-(3,4-Diethyl-1,2,3,4-tetrahydrochinolin-2-on-6-yl)-3,4-diaza-bicyclo[4.2.0]octen-(2)-on-(5)
2-(3-Ethyl-4-methyl-1,2,3,4-tetrahydrochinolin-2-on-6-yl)-3,4-diaza-bicyclo[4.2.0]octen-(2)-on-(5)
2-(4-Ethyl-3-methyl-1,2,3,4-tetrahydrochinolin-2-on-6-yl)-3,4-diaza-bicyclo[4.2.0]octen-(2)-on-(5)
2-(3,3,4,4-Tetramethyl-1,2,3,4-tetrahydrochinolin-2-on-6-yl)-3,4-diaza-bicyclo[4.2.0]octen-(2)-on-(5)
2-(3,4-Diethyl-3,4-dimethyl-1,2,3,4-tetrahydrochinolin-2-on-6-yl)-3,4-diaza-bicyclo[4.2.0]octen-(2)-on-(5)
2-(1,3-Dimethyl-1,2,3,4-tetrahydrochinolin-2-on-6-yl)-3,4-diaza-bicyclo[4.2.0]octen-(2)-on-(5)
2-(1,3,4-Trimethyl-1,2,3,4-tetrahydrochinolin-2-on-6-yl)-3,4-diaza-bicyclo[4.2.0]octen-(2)-on-(5)
2-(1,3,3-Trimethyl-1,2,3,4-tetrahydrochinolin-2-on-6-yl)-3,4-diaza-bicyclo[4.2.0]octen-(2)-on-(5)
2-(1,4,4-Trimethyl-1,2,3,4-tetrahydrochinolin-2-on-6-yl)-3,4-diaza-bicyclo[4.2.0]octen-(2)-on-(5)
2-(1,2,3,4-Tetrahydrochinolin-2-on-6-yl)-4-methyl-3,4-diaza-bicyclo[4.2.0]octen-(2)-on-(5)
2-(1-Methyl-1,2,3,4-tetrahydrochinolin-2-on-6-yl)-4-methyl-3,4-diaza-bicyclo[4.2.0]octen-(2)-on-(5)
2-(1,3-Dimethyl-1,2,3,4-tetrahydrochinolin-2-on-6-yl)-4-methyl-3,4-diaza-bicyclo[4.2.0]octen-(2)-on-(5)

2-(1,3,4-Trimethyl-1,2,3,4-tetrahydrochinolin-2-on-6-yl)-
4-methyl-3,4-diaza-bicyclo[4.2.0]octen-(2)-on-(5)
2-(1,2,3,4-Tetrahydrochinolin-2-on-6-yl)-3,4-diaza-bicyclo[4.1.0]hepten-(2)-on-(5)
2-(1-Methyl-1,2,3,4-tetrahydrochinolin-2-on-6-yl)-3,4-diaza-bicyclo[4.1.0]hepten-(2)-on-(5)
2-(1-Ethyl-1,2,3,4-tetrahydrochinolin-2-on-6-yl)-3,4-diaza-bicyclo[4.1.0]hepten-(2)-on-(5)
2-(1-Propyl-1,2,3,4-tetrahydrochinolin-2-on-6-yl)-3,4-diaza-bicyclo[4.1.0]hepten-(2)-on-(5)
2-(3-Methyl-1,2,3,4-tetrahydrochinolin-2-on-6-yl)-3,4-diaza-bicyclo[4.1.0]hepten-(2)-on-(5)
2-(3-Ethyl-1,2,3,4-tetrahydrochinolin-2-on-6-yl)-3,4-diaza-bicyclo[4.1.0]hepten-(2)-on-(5)
2-(4-Methyl-1,2,3,4-tetrahydrochinolin-2-on-6-yl)-3,4-diaza-bicyclo[4.1.0]hepten-(2)-on-(5)
2-(4-Ethyl-1,2,3,4-tetrahydrochinolin-2-on-6-yl)-3,4-diaza-bicyclo[4.1.0]hepten-(2)-on-(5)
2-(3,3-Dimethyl-1,2,3,4-tetrahydrochinolin-2-on-6-yl)-3,4-diaza-bicyclo[4.1.0]hepten-(2)-on-(5)
2-(3,3-Diethyl-1,2,3,4-tetrahydrochinolin-2-on-6-yl)-3,4-diaza-bicyclo[4.1.0]hepten-(2)-on-(5)
2-(3,4-Dimethyl-1,2,3,4-tetrahydrochinolin-2-on-6-yl)-3,4-diaza-bicyclo[4.1.0]hepten-(2)-on-(5)
2-(3,4-Diethyl-1,2,3,4-tetrahydrochinolin-2-on-6-yl)-3,4-diaza-bicyclo[4.1.0]hepten-(2)-on-(5)
2-(3-Ethyl-4-methyl-1,2,3,4-tetrahydrochinolin-2-on-6-yl)-
3,4-diaza-bicyclo[4.1.0]hepten-(2)-on-(5)
2-(4-Ethyl-3-methyl-1,2,3,4-tetrahydrochinolin-2-on-6-yl)-
3,4-diaza-bicyclo[4.1.0]hepten-(2)-on-(5)
2-(3,3,4,4-Tetramethyl-1,2,3,4-tetrahydrochinolin-2-on-6-yl)-
3,4-diaza-bicyclo[4.1.0]hepten-(2)-on-(5)
2-(3,4-Diethyl-3,4-dimethyl-1,2,3,4-tetrahydrochinolin-2-on-6-yl)-
3,4-diaza-bicyclo[4.1.0]hepten-(2)-on-(5)
2-(1,3-Dimethyl-1,2,3,4-tetrahydrochinolin-2-on-6-yl)-3,4-diaza-bicyclo[4.1.0]hepten-(2)-on-(5)
2-(1,4-Dimethyl-1,2,3,4-tetrahydrochinolin-2-on-6-yl)-3,4-diaza-bicyclo[4.1.0]hepten-(2)-on-(5)
2-(1,3,4-Trimethyl-1,2,3,4-tetrahydrochinolin-2-on-6-yl)-
3,4-diaza-bicyclo[4.1.0]hepten-(2)-on-(5)
2-(1,3,3-Trimethyl-1,2,3,4-tetrahydrochinolin-2-on-6-yl)-
3,4-diaza-bicyclo[4.1.0]hepten-(2)-on-(5)
2-(1,4,4-Trimethyl-1,2,3,4-tetrahydrochinolin-2-on-6-yl)-
3,4-diaza-bicyclo[4.1.0]hepten-(2)-on-(5)
2-(1,2,3,4-Tetrahydrochinolin-2-on-6-yl)-4-methyl-3,4-diaza-bicyclo[4.1.0]hepten-(2)-on-(5)
2-(1-Methyl-1,2,3,4-tetrahydrochinolin-2-on-6-yl)-
4-methyl-3,4-diaza-bicyclo[4.1.0]hepten-(2)-on-(5)
2-(1,3-Dimethyl-1,2,3,4-tetrahydrochinolin-2-on-6-yl)-
4-methyl-3,4-diaza-bicyclo[4.1.0]hepten-(2)-on-(5)
2-(1,3,4-Trimethyl-1,2,3,4-tetrahydrochinolin-2-on-6-yl)-
4-methyl-3,4-diaza-bicyclo[4.1.0]hepten-(2)-on-(5)
2-(1,3,3-Trimethyl-1,2,3,4-tetrahydrochinolin-2-on-6-yl)-
4-methyl-3,4-diaza-bicyclo[4.1.0]hepten-(2)-on-(5)
2-(1,4-Dimethyl-1,2,3,4-tetrahydrochinolin-2-on-6-yl)-
4-methyl-3,4-diaza-bicyclo[4.1.0]hepten-(2)-on-(6)
2-(2,3,4,5-Tetrahydro-benzo[b]azepin-2-(1H)-on-7-yl)-3,4-diaza-bicyclo[4.3.0]nonen-(2)-on-(5)
2-(1-Methyl-2,3,4,5-tetrahydro-benzo[b]azepin-2-(1H)-on-7-yl)-
3,4-diaza-bicyclo[4.3.0]nonen-(2)-on-(5)
2-(3-Methyl-2,3,4,5-tetrahydro-benzo[b]azepin-2-(1H)-on-7-yl)-
3,4-diaza-bicyclo[4.3.0]nonen-(2)-on-(5)
2-(4-Methyl-2,3,4,5-tetrahydro-benzo[b]azepin-2-(1H)-on-7-yl)-
3,4-diaza-bicyclo[4.3.0]nonen-(2)-on-(5)
2-(5-Methyl-2,3,4,5-tetrahydro-benzo[b]azepin-2-(1H)-on-7-yl)-
3,4-diaza-bicyclo[4.3.0]nonen-(2)-on-(5)
2-(3,4-Dimethyl-2,3,4,5-tetrahydro-benzo[b]azepin-2-(1H)-on-7-yl)-
3,4-diaza-bicyclo[4.3.0]nonen-(2)-on-(5)
2-(3,5-Dimethyl-2,3,4,5-tetrahydro-benzo[b]azepin-2-(1H)-on-7-yl)-
3,4-diaza-bicyclo[4.3.0]nonen-(2)-on-(5)
2-(4,5-Dimethyl-2,3,4,5-tetrahydro-benzo[b]azepin-2-(1H)-on-7-yl)-
3,4-diaza-bicyclo[4.3.0]nonen-(2)-on-(5)
2-(1,3-Dimethyl-2,3,4,5-tetrahydro-benzo[b]azepin-2-(1H)-on-7-yl)-
3,4-diaza-bicyclo[4.3.0]nonen-(2)-on-(5)
2-(1,4-Dimethyl-2,3,4,5-tetrahydro-benzo[b]azepin-2-(1H)-on-7-yl)-
3,4-diaza-bicyclo[4.3.0]nonen-(2)-on-(5)
2-(1,5-Dimethyl-2,3,4,5-tetrahydro-benzo[b]azepin-2-(1H)-on-7-yl)-
3,4-diaza-bicyclo[4.3.0]nonen-(2)-on-(5)
2-(1,3,4-Trimethyl-2,3,4,5-tetrahydro-benzo[b]azepin-2-(1H)-on-7-yl)-
3,4-diaza-bicyclo[4.3.0]nonen-(2)-on-(5)
2-(1,3,5-Trimethyl-2,3,4,5-tetrahydro-benzo[b]azepin-2-(1H)-on-7-yl)-3,4-diaza-bicyclo[4.3.0]nonen-(2)-on-(5)
2-(1,4,5-Trimethyl-2,3,4,5-tetrahydro-benzo[b]azepin-2-(1H)-on-7-yl)-3,4-diaza-bicyclo[4.3.0]nonen-(2)-on-(5)
2-(3,4,5-Trimethyl-2,3,4,5-tetrahydro-benzo[b]azepin-2-(1H)-on-7-yl)-3,4-diaza-bicyclo[4.3.0]nonen-(2)-on-(5)
2-(1,3,4,5-Tetramethyl-2,3,4,5-tetrahydro-benzo[b]azepin-2-(1H)-on-7-yl)-3,4-diaza-bicyclo[4.3.0]nonen-(2)-on-(5)
2-(2,3,4,5-Tetrahydro-benzo[b]azepin-2-(1H)-on-7-yl)-4-methyl-3,4-diaza-bicyclo[4.3.0]nonen-(2)-on-(5)
2-(1-Methyl-2,3,4,5-tetrahydro-benzo[b]azepin-2-(1H)-on-7-yl)-4-methyl-3,4-diaza-bicyclo[4.3.0]nonen-(2)-on-(5)
2-(1,3-Dimethyl-2,3,4,5-tetrahydro-benzo[b]azepin-2-(1H)-on-7-yl)-4-methyl-3,4-diaza-bicyclo[4.3.0]nonen-(2)-on-(5)
2-(1,4-Dimethyl-2,3,4,5-tetrahydro-benzo[b]azepin-2-(1H)-on-7-yl)-4-methyl-3,4-diaza-bicyclo[4.3.0]nonen-(2)-on-(5)
2-(2,3,4,5-Tetrahydro-benzo[b]azepin-2-(1H)-on-7-yl)-3,4-diaza-bicyclo[4.3.0]octen-(2)-on-(5)
2-(1-Methyl-2,3,4,5-tetrahydro-benzo[b]azepin-2-(1H)-on-7-yl)-3,4-diaza-bicyclo[4.2.0]octen-(2)-on-(5)
2-(3-Methyl-2,3,4,5-tetrahydro-benzo[b]azepin-2-(1H)-on-7-yl)-3,4-diaza-bicyclo[4.2.0]octen-(2)-on-(5)
2-(4-Methyl-2,3,4,5-tetrahydro-benzo[b]azepin-2-(1H)-on-7-yl)-3,4-diaza-bicyclo[4.3.0]octen-(2)-on-(5)
2-(5-Methyl-2,3,4,5-tetrahydro-benzo[b]azepin-2-(1H)-on-7-yl)-3,4-diaza-bicyclo[4.2.0]octen-(2)-on-(5)
2-(3,4-Dimethyl-2,3,4,5-tetrahydro-benzo[b]azepin-2-(1H)-on-7-yl)-3,4-diaza-bicyclo[4.2.0]octen-(2)-on-(5)
2-(3,5-Dimethyl-2,3,4,5-tetrahydro-benzo[b]azepin-2-(1H)-on-7-yl)-3,4-diaza-bicyclo[4.2.0]octen-(2)-on-(5)
2-(4,5-Dimethyl-2,3,4,5-tetrahydro-benzo[b]azepin-2-(1H)-on-7-yl)-3,4-diaza-bicyclo[4.2.0]octen-(2)-on-(5)
2-(1,3-Dimethyl-2,3,4,5-tetrahydro-benzo[b]azepin-2-(1H)-on-7-yl)-3,4-diaza-bicyclo[4.2.0]octen-(2)-on-(5)
2-(1,4-Dimethyl-2,3,4,5-tetrahydro-benzo[b]azepin-2-(1H)-on-7-yl)-3,4-diaza-bicyclo[4.2.0]octen-(2)-on-(5)
2-(1,5-Dimethyl-2,3,4,5-tetrahydro-benzo[b]azepin-2-(1H)-on-7-yl)-3,4-diaza-bicyclo[4.2.0]octen-(2)-on-(5)
2-(1,3,4-Trimethyl-2,3,4,5-tetrahydro-benzo[b]azepin-2-(1H)-on-7-yl)-3,4-diaza-bicyclo[4.2.0]octen-(2)-on-(5)
2-(1,3,5-Trimethyl-2,3,4,5-tetrahydro-benzo[b]azepin-2-(1H)-on-7-yl)-3,4-diaza-bicyclo[4.2.0]octen-(2)-on-(5)
2-(1,4,5-Trimethyl-2,3,4,5-tetrahydro-benzo[b]azepin-2-(1H)-on-7-yl)-3,4-diaza-bicyclo[4.2.0]octen-(2)-on-(5)
2-(3,4,5-Trimethyl-2,3,4,5-tetrahydro-benzo[b]azepin-2-(1H)-on-7-yl)-3,4-diaza-bicyclo[4.2.0]octen-(2)-on-(5)
2-(1,3,4,5-Tetramethyl-2,3,4,5-tetrahydro-benzo[b]azepin-2-(1H)-on-7-yl)-3,4-diaza-bicyclo[4.2.0]octen-(2)-on-(5)
2-(2,3,4,5-Tetrahydro-benzo[b]azepin-2-(1H)-on-7-yl)-4-methyl-3,4-diaza-bicyclo[4.2.0]octen-(2)-on-(5)
2-(1-Methyl-2,3,4,5-tetrahydro-benzo[b]azepin-2-(1H)-on-7-yl)-4-methyl-3,4-diaza-bicyclo[4.2.0]octen-(2)-on-(5)
2-(1,3-Dimethyl-2,3,4,5-tetrahydro-benzo[b]azepin-2-(1H)-on-7-yl)-4-methyl-3,4-diaza-bicyclo[4.2.0]octen-(2)-on-(5)
2-(1,4-Dimethyl-2,3,4,5-tetrahydro-benzo[b]azepin-2-(1H)-on-7-yl)-4-methyl-3,4-diaza-bicyclo[4.2.0]octen-(2)-on-(5)
2-(2,3,4,5-Tetrahydro-benzo[b]azepin-2-(1H)-on-7-yl)-3,4-diaza-bicyclo[4.1.0]hepten-(2)-on-(5)
2-(1-Methyl-2,3,4,5-tetrahydro-benzo[b]azepin-2-(1H)-on-7-yl)-3,4-diaza-bicyclo[4.1.0]hepten-(2)-on-(5)
2-(3-Methyl-2,3,4,5-tetrahydro-benzo[b]azepin-2-(1H)-on-7-yl)-3,4-diaza-bicyclo[4.1.0]hepten-(2)-on-(5)
2-(4-Methyl-2,3,4,5-tetrahydro-benzo[b]azepin-2-(1H)-on-7-yl)-3,4-diaza-bicyclo[4.1.0]hepten-(2)-on-(5)
2-(5-Methyl-2,3,4,5-tetrahydro-benzo[b]azepin-2-(1H)-on-7-yl)-3,4-diaza-bicyclo[4.1.0]hepten-(2)-on-(5)
2-(3,4-Dimethyl-2,3,4,5-tetrahydro-benzo[b]azepin-2-(1H)-on-7-yl)-3,4-diaza-bicyclo[4.1.0]hepten-(2)-on-(5)

2-(3,5-Dimethyl-2,3,4,5-tetrahydro-benzo[b]azepin-2-(1H)-on-7-yl)-3,4-diaza-bicyclo[4.1.0]hepten-(2)-on-(5)
2-(4,5-Dimethyl-2,3,4,5-tetrahydro-benzo[b]azepin-2-(1H)-on-7-yl)-3,4-diaza-bicyclo[4.1.0]hepten-(2)-on-(5)
2-(1,3-Dimethyl-2,3,4,5-tetrahydro-benzo[b]azepin-2-(1H)-on-7-yl)-3,4-diaza-bicyclo[4.1.0]hepten-(2)-on-(5)
2-(1,4-Dimethyl-2,3,4,5-tetrahydro-benzo[b]azepin-2-(1H)-on-7-yl)-3,4-diaza-bicyclo[4.1.0]hepten-(2)-on-(5)
2-(1,5-Dimethyl-2,3,4,5-tetrahydro-benzo[b]azepin-2-(1H)-on-7-yl)-3,4-diaza-bicyclo[4.1.0]hepten-(2)-on-(5)
2-(1,3,4-Trimethyl-2,3,4,5-tetrahydro-benzo[b]azepin-2-(1H)-on-7-yl)-3,4-diaza-bicyclo[4.1.0]hepten-(2)-on-(5)
2-(1,3,5-Trimethyl-2,3,4,5-tetrahydro-benzo[b]azepin-2-(1H)-on-7-yl)-3,4-diaza-bicyclo[4.1.0]hepten-(2)-on-(5)
2-(1,4,5-Trimethyl-2,3,4,5-tetrahydro-benzo[b]azepin-2-(1H)-on-7-yl)-3,4-diaza-bicyclo[4.1.0]hepten-(2)-on-(5)
2-(3,4,5-Trimethyl-2,3,4,5-tetrahydro-benzo[b]azepin-2-(1H)-on-7-yl)-3,4-diaza-bicyclo[4.1.0]hepten-(2)-on-(5)
2-(1,3,4,5-Tetramethyl-2,3,4,5-tetrahydro-benzo[b]azepin-2-(1H)-on-7-yl)-3,4-diaza-bicyclo[4.1.0]hepten-(2)-on-(5)
2-(2,3,4,5-Tetrahydro-benzo[b]azepin-2-(1H)-on-7-yl)-4-methyl-3,4-diaza-bicyclo[4.1.0]hepten-(2)-on-(5)
2-(1-Methyl-2,3,4,5-tetrahydro-benzo[b]azepin-2-(1H)-on-7-yl)-4-methyl-3,4-diaza-bicyclo[4.1.0]hepten-(2)-on-(5)
2-(1,3-Dimethyl-2,3,4,5-tetrahydro-benzo[b]azepin-2-(1H)-on-7-yl)-4-methyl-3,4-diaza-bicyclo[4.1.0]hepten-(2)-on-(5)
2-(1,4-Dimethyl-2,3,4,5-tetrahydro-benzo[b]azepin-2-(1H)-on-7-yl)-4-methyl-3,4-diaza-bicyclo[4.1.0]hepten-(2)-on-(5)

Es sei darauf hingewiesen, daß die erfindungsgemäßen Verbindungen der Formel I in den Stellungen 1 und 6 des 3,4-Diaza-bicyclo-alkenon-Ringes asymmetrische Kohlenstoffatome aufweisen und in der 3-Position des Indolinons bzw. der 3- oder 4-Position des Tetrahydrochinolinon- bzw. der 3-, 4- oder 5-Position des Tetrahydrobenzo[b]azepinonringes asymmetrische Kohlenstoffatome aufweisen können und als Racemate erhalten werden. Diese können in bekannter Weise in die optischen Isomeren getrennt werden.

Die erfindungsgemäßen Verbindungen der Formel I zeichnen sich durch eine starke thrombozytenaggregationshemmende Wirkung und durch eine starke blutdrucksenkende Wirkung aus. Sie sind demnach als Antihypertensiva und zur Prophylaxe und Therapie thromboembolischer Erkrankungen geeignet.

Zur Untersuchung der pharmakodynamischen Eigenschaften der erfindungsgemäßen Produkte wurden folgende Methoden verwendet:

1. Hemmung der durch Collagen induzierten Thrombozytenaggregation an der Ratte

Die Substanzen wurden Gruppen von 10—15 männlichen Sprague-Dawley-Ratten (200—250 g) oral appliziert. 1 h nach der Applikation wurde in Ether-Narkose Blut entnommen und durch Zentrifugation (300 g, 10 min Dauer bei 4°C) thrombozytenreiches Plasma gewonnen. Die photometrische Messung der Thrombozytenaggregation erfolgte unter Zusatz von $MgCl_2$ (Endkonzentration 10 mmol/l) und von Collagen Stago (Endkonzentration 0,02 mg/ml) im Born-Aggregometer Mk 3. Als Aggregationsmaß fand die maximale Extinktionsänderung/sec Verwendung.

Als ED 33% wurde die Dosis bestimmt, welche die durch Collagen induzierte Thrombozytenaggregation um 33% hemmte.

2. Antihypertensive Wirkung an der spontan hypertonen Ratte (SHR)

Die Substanzen wurden männlichen spontan hypertonen Okamoto-Ratten (4—8 Tiere/Dosis, Gewicht: 270—360 g) oral appliziert. Der systolische Blutdruck wurde vor und 2 h nach der Applikation unblutig am Schwanz mit Hilfe von Piezokristallaufnehmern ermittelt.

Als ED 20% wurde unter Berücksichtigung der Werte unbehandelter Kontrolltiere die Dosis bestimmt, welche den systolischen Druck um 20% senkte.

3. Akute Toxizität an der Maus

Zur Bestimmung der akuten Toxizität wurden die Substanzen Gruppen von je 5—10 weiblichen NMRI-Mäusen (Gewicht 20—23 g) intraperitoneal appliziert und die Todeshäufigkeit beobachtet.

Die erfindungsgemäßen Verbindungen zeichnen sich bei geringer Toxizität durch starke thrombozytenaggregationshemmende (Beispiel C, E, F und H) und durch antihypertensive Effekte (Beispiel F und H) aus (Tabelle 1). Unerwartet und besonders hervorzuheben ist die im Vergleich mit den bekannten Referenzsubstanzen I (6-(p-Propoxyphenyl)-4,5-dihydro-3(2H)-pyridazinon; DE-OS 2 207 517) und II (6,6-(5-methyl-3-oxo-2,3,4,5-tetrahydropyridazin-6-yl)-1,2,3,4-tetrahydrochinolin-2-on; DE-OS 2 845 220) hohe Spezifität der thrombozytenaggregationshemmenden Wirkung, die durch den Unterschied zwischen den aggregationshemmenden und den antihypertensiv wirksamen Dosen (Q, siehe Tabelle 1) quantifiziert werden kann und die pharmakotherapeutische Verwendbarkeit der erfindungsgemäßen Verbindungen begünstigt.

Tabelle 1

| Beispiel | Thrombozytenaggregationshemmung ED 33% [1] | Antihypertensive Wirkung ED 20% [1] | Q [2] | Todeshäufigkeit [3] x/n |
|---|---|---|---|---|
| F | 0,18 | 2,2 | >12 | 1/5 |
| C | 1,3 | >10 | >7,7 | 3/5 |
| E | 0,87 | >10 | >11 | 0/5 |
| H | 0,33 | 4,0 | 12 | 0/5 |
| I | 0,63 | 1,2 | 1,9 | 10/10 |
| II | 0,23 | 0,79 | 3,4 | 2/5 |

[1] Ratte per os, mg/kg
[2] ED 20%/ED 33%
[3] Maus i. p. 1000 mg/kg

Gegenstand der vorliegenden Erfindung sind demnach auch Arzneimittel, die eine Verbindung der Formel I enthalten.

Die erfindungsgemäßen Verbindungen können in üblicher Weise oral oder parenteral (intravenös, intramuskulär, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen etwa 0,1 und 10 mg/kg Körpergewicht bei parenteraler Gabe. Im Normalfall werden mit täglichen Dosen von 5 bis 50 mg/kg oral und 0,5 bis 5 mg/kg parenteral zufriedenstellende Ergebnisse erzielt.

Die neuen Verbindungen können in den gebräuchlichen galenischen Applikationsformen fest oder flüssig angewendet werden, z. B. als Tabletten, Filmtabletten, Kapseln, Pulver, Granulate, Dragees oder Suppositorien. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker/Pharmazeutische Technologie, Thieme-Verlag, Stuttgart, 1978). Die so erhaltenen Wirkstoffe enthalten den Wirkstoff normalerweise in einer Menge von 1 und 99 Gewichtsprozent.

## Herstellung der Ausgangsverbindungen

a) Zu 120 g (0,9 mol) wasserfreiem Aluminiumchlorid gibt man unter Rühren innerhalb weniger Minuten tropfenweise 20 ml (0,26 mol) Dimethylformamid, wobei eine stark exotherme Reaktion eintritt. Man fügt dann bei 140° C portionsweise ein Gemisch aus 11,8 g (0,089 mol) Indolinon-2 und 11,3 g (0,09 mol) Cyclobutandicarbonsäureanhydrid zu und rührt anschließend noch 10 min bei 140° C nach. Die Schmelze wird nun in 0,5 kg Eis eingetragen. Der ausgefallene Festkörper wird abgesaugt und die Wasserphase mehrmals mit Essigester extrahiert. Man erhält 21,7 g (93,6%) cis-2(Indolin-2-on-5-oyl)cyclobutancarbonsäure, Fp. = 211 bis 212° C.

Analog erhält man:

b) cis-2-(1-Methyl-indolin-2-on-5-oyl)cyclobutancarbonsäure, Fp. 210 bis 212° C, Ausbeute 84%.
c) cis-2-(Indolin-2-on-5-oyl)cyclopropancarbonsäure, Fp. 187 bis 190° C, Ausbeute 81%.
d) cis-2-(1-Methylindolin-2-on-5-oyl)cyclopropancarbonsäure, Fp. 237 bis 240° C, Ausbeute 81,5%.
e) cis-2-(1,2,3,4-Tetrahydrochinolin-2-on-6-oyl)cyclobutancarbonsäure, Fp. 155 bis 158° C, Ausbeute 75%.
f) cis-2-(1,2,3,4-Tetrahydrochinolin-2-on-6-oyl)cyclopropancarbonsäure, Fp. 190 bis 194° C, Ausbeute 57%.
g) cis-2-(2,3,4,5-Tetrahydro-benzo[b]azepin-2-(1H)-on-7-oyl)cyclopropancarbonsäure als hellgelbes Öl, Ausbeute 70%.

Analyse für $C_{15}H_{15}NO_4$ (273):

| | | | |
|---|---|---|---|
| ber. | C 65,9 | H 5,5 | N 5,1 |
| gef. | C 65,9 | H 5,4 | N 4,9 |

Beispiel 1

A) 5,22 g (0,02 mol) cis-2-(Indolin-2-on-5-oyl)cyclobutancarbonsäure werden mit 1,1 g (0,022 mol) Hydrazinhydrat und 50 ml Ethanol 11 Stunden am Rückfluß gehalten. Nach dem Absaugen bei Raumtemperatur und Umkristallisieren aus Dimethylformamid/Wasser erhält man 4,7 g (92%) 2-(Indolin-2-on-5-yl)-3,4-diaza-bicyclo[4.2.0]octen-(2)-on-(5), Fp. 309 bis 312° C.

Analyse für $C_{14}H_{13}N_3O_2$ (255):

| | | | |
|---|---|---|---|
| ber.: | C 65,8 | H 5,1 | N 16,5 |
| gef.: | C 65,5 | H 5,3 | N 16,5 |

Analog erhält man:

B) 2-(1-Methylindolin-2-on-5-yl)-3,4-diaza-bicyclo[4.2.0]-octen-(2)-on-(5), Fp. 256 bis 259° C, Ausbeute 85%.

Analyse für $C_{15}H_{15}N_3O_2$ (269):

| | | | |
|---|---|---|---|
| ber.: | C 66,9 | H 5,6 | N 15,6 |
| gef.: | C 66,6 | H 5,7 | N 15,5 |

C) 2-(Indolin-2-on-5-yl)-3,4-diaza-bicyclo[4.1.0]hepten-(2)-on-(5), Fp. 305 bis 307° C, Ausbeute 81%.

Analyse für $C_{13}H_{11}N_3O_2$ (241):

| | | | |
|---|---|---|---|
| ber.: | C 64,7 | H 4,6 | N 17,4 |
| gef.: | C 64,7 | H 4,6 | N 17,5 |

D) 2-(1-Methylindolin-2-on-5-yl)-3,4-diaza-bicyclo-[4.1.0]hepten-(2)-on-(5), Fp. 265 bis 267° C, Ausbeute 51%.

Analyse für $C_{14}H_{13}N_3O_2$ (255):

| | | | |
|---|---|---|---|
| ber.: | C 65,9 | H 5,1 | N 16,5 |
| gef.: | C 65,4 | H 5,1 | N 17,0 |

E) 2-(1,2,3,4-Tetrahydrochinolin-2-on-6-yl)-3,4-diaza-bicyclo[4.2.0]octen-(2)-on-(5), Fp. 349 bis 353° C, Ausbeute 84%.

Analyse für $C_{15}H_{15}N_3O_2$ (269):

| | | | |
|---|---|---|---|
| ber.: | C 66,9 | H 5,6 | N 15,6 |
| gef.: | C 66,7 | H 5,6 | N 15,5 |

F) 2-(1,2,3,4-Tetrahydrochinolin-2-on-6-yl)-3,4-diaza-bicyclo[4.1.0]hepten-(2)-on-(5), Fp. 310 bis 311° C (Zers.), Ausbeute 75%.

Analyse für $C_{14}H_{13}N_3O_2 \times {}^1/_4\ H_2O$:

| | | | |
|---|---|---|---|
| ber.: | C 64,8 | H 5,2 | N 16,4 |
| gef.: | C 64,7 | H 5,2 | N 16,2 |

G) 2-(1,2,3,4-Tetrahydrochinolin-2-on-6-yl)-4-methyl-3,4-diaza-bicyclo[4.1.0]hepten-(2)-on-(5), Fp. 255 bis 259° C, Ausbeute 76%.

Analyse für $C_{15}H_{15}N_3O_2$ (269):
ber.: C 66,9 H 5,6 N 15,6
gef.: C 66,6 H 5,7 N 15,5

H) 2-(2,3,4,5-Tetrahydro-benzo[b]azepin-2-(1H)-on-7-yl)-3,4-diaza-bicyclo[4.1.0]hepten-(2)-on-(5), Fp. 308 bis 310° C, Ausbeute 75%.

Analyse für $C_{15}H_{15}N_3O_2$ (269):
ber.: C 66,9 H 5,6 N 15,6
gef.: C 66,2 H 5,3 N 15,4

Formulierungsbeispiele

I. Es werden Tabletten folgender Zusammensetzung hergestellt:

| | |
|---|---|
| Wirkstoff | 10 mg |
| Polyvinylpyrrolidon (mittl. M. G. 25 000) | 170 mg |
| Polyethylenglykol (mittl. M. G. 4000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10%iger wäßriger Lösung befeuchtet, durch ein Sieb mit der lichten Maschenweite 1,0 mm getrieben und bei 50° C getrocknet. Dieses Granulat wird mit Polyethylenglykol (mittl. M. G. 4000), Hydroxypropylmethylcellulose, Talkum und Magnesiumstearat vermischt und zu Tabletten à 240 mg verpreßt.

II. Es werden Dragees folgender Zusammensetzung hergestellt:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8%igen wäßrigen Lösung des Polyvinylpyrrolidons durch ein Sieb 1,5 mm granuliert, bei 50° C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

**Patentansprüche**

1. 2-Aryl-3,4-diaza-bicyclo[4.n.0]alken-(2)-one-(5) der Formel I

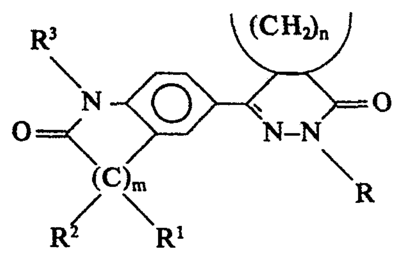

(I)

worin
m und n gleich oder verschieden sind und die Zahlen 1, 2 oder 3 bedeuten, und
R, $R^1$, $R^2$ und $R^3$ gleich oder verschieden sind und Wasserstoffatome oder Alkylgruppen mit 1 bis 6 C-Atomen bedeuten.

2. 2-(Indolin-2-on-5-yl)-3,4-diaza-bicyclo[4.1.0]hepten-(2)-on-(5).

3. 2-(1,2,3,4-Tetrahydrochinolin-2-on-6-yl)-3,4-diaza-bicyclo[4.2.0]octen-(2)-on-(5).

4. 2-(1,2,3,4-Tetrahydrochinolin-2-on-6-yl)-3,4-diaza-bicyclo[4.1.0]heptan-(2)-on-(5).

5. 2-(2,3,4,5-Tetrahydro-benzo[b]azepin-2-(1H)-on-7-yl)-3,4-diaza-bicyclo[4.1.0]hepten-(2)-on-(5).

6. Verfahren zur Herstellung der Verbindung der Formel I gemäß Anspruch 1, dadurch gekennzeichnet, daß man eine Cycloalkancarbonsäure der Formel II

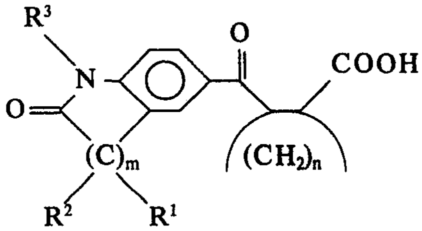

(II)

in der $R^1$, $R^2$ und $R^3$ die oben angegebenen Bedeutungen haben, mit einem Hydrazin der Formel $NH_2NHR$, in der R die oben angegebene Bedeutung hat, umsetzt.

7. Arzneimittel enthaltend eine Verbindung der Formel I gemäß Anspruch 1.

8. Verbindung der Formel I gemäß Anspruch 1 zur Verwendung bei der Bekämpfung von Bluthochdruck und thrombo-embolischer Erkrankungen.

## Claims

1. A 2-aryl-3,4-diazabicyclo[4.n.0]alk-2-en-5-one of the formula I

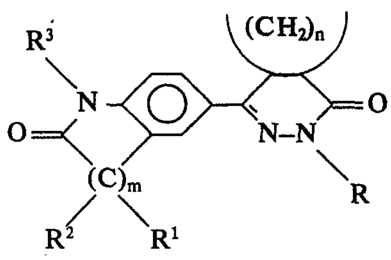

(I)

where m and n are identical or different and each is 1, 2 or 3 and R, $R^1$, $R^2$ and $R^3$ are identical or different and each is hydrogen or alkyl of 1 to 6 C atoms.

2. 2-(Indolin-2-on-5-yl)-3,4-diazabicyclo[4.1.0]hept-2-en-5-one.

3. 2-(1,2,3,4-Tetrahydroquinolin-2-on-6-yl)-3,4-diazabicyclo[4.2.0]oct-2-en-5-one.

4. 2-(1,2,3,4-Tetrahydroquinolin-2-on-6-yl)-3,4-diazabicyclo[4.1.0]hept-2-en-5-one.

5. 2-(2,3,4,5-Tetrahydro-benzo[b]azepin-2-(1H)-on-7-yl)-3,4-diazabicyclo[4.1.0]hept-2-en-5-one.

6. A process for the preparation of a compound of the formula I as claimed in claim 1, wherein a cycloalkanecarboxylic acid of the formula II

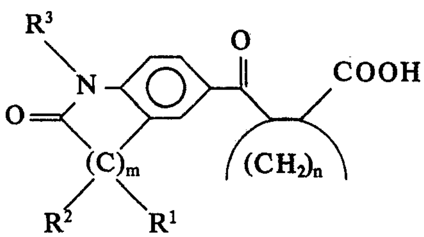

(II)

where $R^1$, $R^2$ and $R^3$ have the above meanings, is reacted with a hydrazine of the formula $NH_2NHR$, where R has the above meanings.

7. A drug containing a compound of the formula I as claimed in claim 1.

8. A compound of the formula I as claimed in claim 1 for use for controlling high blood pressure and thromboembolic disorders.

**Revendications**

1. 2-aryl-3,4-diaza-bicyclo[4.n.0]alcen-(2)-ones-(5) de formule I

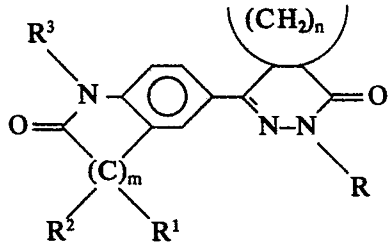

(I)

dans laquelle
m et n sont identiques ou différents et représentent les nombres 1, 2 ou 3, et
R, $R^1$, $R^2$ et $R^3$ sont identiques ou différents et représentent des atomes d'hydrogène ou des groupes alkyle ayant 1 à 6 atomes-C.

2. 2-(Indolin-2-on-5-yl)-3,4-diaza-bicyclo[4.1.0]hepten-(2)-one-(5).

3. 2-(1,2,3,4-Tetrahydrochinolin-2-on-6-yl)-3,4-diaza-bicyclo[4.2.0]octen-(2)-one-(5).

4. 2-(1,2,3,4-Tetrahydrochinolin-2-on-6-yl)-3,4-diaza-bicyclo[4.1.0]hepten-(2)-one-(5).

5. 2-(2,3,4,5-Tetrahydro-benzo[b]azepin-2-(1H)-on-7-yl)-3,4-diaza-bicyclo[4.1.0]hepten-(2)-one-(5).

6. Procédé de préparation de composés de formule I selon la revendication 1, caractérisé par le fait qu'on fait réagir un acide cycloalcancarboxylique de formule II

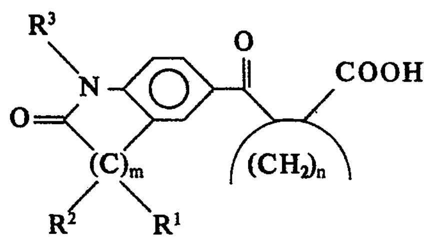

(II)

dans laquelle $R^1$, $R^2$ et $R^3$ ont les significations indiquées plus haut, avec une hydrazine de formule $NH_2NHR$, dans laquelle R a la signification donnée plus haut.

7. Médicament contenant un composé de formule I selon la revendication 1.

8. Composé de formule I selon la revendication 1 pour utilisation dans la lutte contre l'hypertension du sang et les maladies thrombo-emboliques.